# EUROPEAN PATENT APPLICATION

(11) **EP 4 621 064 A1**
(43) Date of publication of application: **24.09.2025**
(21) Application number: 23891384.2
(22) Date of filing: 02.11.2023
(51) Int. Cl.: C12Q 1/02, C12M 1/00, G01N 21/17, G01N 33/483, G06T 7/00, G16B 40/20

(54) **EMBRYO CLASSIFICATION METHOD, COMPUTER PROGRAM, AND EMBRYO CLASSIFICATION APPARATUS**

(30) Priority: 16.11.2022 JP 2022183037
(71) Applicant: SCREEN Holdings Co., Ltd., Kyoto-shi, Kyoto 602-8585 (JP)
(72) Inventor: HASEBE Ryo, Kyoto-shi, Kyoto 602-8585 (JP)
(74) Representative: Goddar, Heinz J.
(86) International application number: PCT/JP2023/039711
(87) International publication number: WO 2024/106231

(57) **Abstract**

First, a three-dimensional image (D2) of a blastocyst embryo is acquired by optical coherence tomography. Secondly, a parameter (P) representing at least one of a volume of an inner cell mass, a thickness of an inner cell mass, a thickness distribution of an inner cell mass, a volume of a trophectoderm, a thickness of a trophectoderm, a thickness distribution of a trophectoderm, or the number of cells in a trophectoderm is calculated on the basis of the three-dimensional image (D2). Then, the parameter (P) of a blastocyst embryo to be classified is input to a trained machine learning model (M1, M2, M3) that has the parameter (P) as an input variable and has a classification result of the blastocyst embryo as an output variable, and the blastocyst embryo is classified on the basis of a classification result output from the machine learning model. Thus, classification of the blastocyst embryo can be performed quantitatively on the basis of the three-dimensional image (D2).

## Description

### Technical Field

The present invention relates to an embryo classification method, a computer program, and an embryo classification apparatus.

### Background Art

In an assisted reproductive technology procedure aimed at fertility treatment, an embryo fertilized outside a body is cultured for a certain period of time, and then is returned back into the body. In such an assisted reproductive technology procedure, it is important to properly evaluate states of multiple embryos and return a good embryo back into a body in order to increase a success rate of pregnancy.

An embryo, after fertilization, goes through a cleavage stage and reaches a blastocyst stage. An embryo at the blastocyst stage (hereinafter referred to as an "blastocyst embryo") includes a hollow spherical zona pellucida, and an inner cell mass and a trophectoderm that are enclosed in the zona pellucida. Conventionally, for evaluation of blastocyst embryos, Gardner's classification is mainly used. In Gardner's classification, a development stage of a blastocyst embryo is classified into six stages, a state of an inner cell mass is classified into three stages, and a state of a trophectoderm is classified into three stages. In a conventional assisted reproductive technology procedure, an embryologist evaluates a blastocyst embryo according to criteria of Gardner's classification while viewing a microscopic image of the embryo.

However, a microscopic image conventionally used by an embryologist in evaluation is a two-dimensional image showing an embryo as seen from one direction, and hence, it is difficult to accurately grasp a three-dimensional structure of the embryo. Then, in recent years, it has been proposed to take an image of an embryo by optical coherence tomography (OCT) and use the thus acquired three-dimensional image for observation of the embryo. A conventional technology for taking an image of an embryo by optical coherence tomography is described in, for example, Patent Literature 1.

### Citation List

### Patent Literature

Patent Literature 1: Japanese Patent Application Laid-Open No. 2019-133429

### Summary of Invention

### Technical Problem

In this regard, however, there has not yet been a well-established method for quantitatively classifying a state of a blastocyst embryo on the basis of a three-dimensional image acquired by optical coherence tomography. With such subjective determination made by an embryologist as described above, determination varies depending on skills of an embryologist in some cases. In order to further increase a success rate of an assisted reproductive technology procedure, there is a demand for a technology that enables quantitative classification of a state of a blastocyst embryo based on a three-dimensional image without depending on skills of an embryologist.

The present invention has been made in view of the above-described situation, and it is an object to provide a technology that enables quantitative classification of a blastocyst embryo based on a three-dimensional image.

### Solution to Problem

To solve the above-described problem, the first invention of the present application is directed to an embryo classification method for classifying a blastocyst embryo, including: a) an image acquisition step of acquiring a three-dimensional image of the blastocyst embryo by optical coherence tomography; b) a parameter calculation step of calculating a parameter representing at least one of a volume of an inner cell mass, a thickness of the inner cell mass, a thickness distribution of the inner cell mass, a volume of a trophectoderm, a thickness of the trophectoderm, a thickness distribution of the trophectoderm, or the number of cells in the trophectoderm, on the basis of the three-dimensional image; and c) a classification step of inputting the parameter of the blastocyst embryo to be classified, to a trained machine learning model that has the parameter as an input variable and has a classification result of the blastocyst embryo as an output variable, and classifying the blastocyst embryo on the basis of the classification result output from the machine learning model.

The second invention of the present application is directed to the embryo classification method of the first invention, wherein the classification result includes an evaluation result of the inner cell mass.

The third invention of the present application is directed to the embryo classification method of the first invention or the second invention, wherein the classification result includes an evaluation result of the trophectoderm.

The fourth invention of the present application is directed to the embryo classification method of any of the first invention to the third invention, wherein the classification result includes an estimation result of a development stage of the blastocyst embryo.

The fifth invention of the present application is directed to the embryo classification method of any of the first invention to the fourth invention, wherein the input variable includes at least one of an average value, a mean value, a standard deviation, a minimum value, a maximum value, or a range, of the thickness of the inner cell mass, as the parameter representing the thickness of the inner cell mas.

The sixth invention of the present application is directed to the embryo classification method of any of the first invention to the fifth invention, wherein the input variable includes at least one of kurtosis, skewness, a mode value, or a density of each bin of a histogram, of the thickness distribution of the inner cell mass, as the parameter representing the thickness distribution of the inner cell mass.

The seventh invention of the present application is directed to the embryo classification method of any of the first invention to the sixth invention, wherein the input variable includes at least one of an average value, a mean value, a standard deviation, a minimum value, a maximum value, or a range, of the thickness of the trophectoderm, as the parameter representing the thickness of the trophectoderm.

The eighth invention of the present application is directed to the embryo classification method of any of the first invention to the seventh invention, wherein the input variable includes at least one of kurtosis, skewness, a mode value, or a density of each bin of a histogram, of the thickness distribution of the trophectoderm, as the parameter representing the thickness distribution of the trophectoderm.

The ninth invention of the present application is directed to the embryo classification method of any of the first invention to the eighth invention, wherein the input variable includes the number of cells, or a cell density, of a part of the trophectoderm not overlapping the inner cell mass, as the parameter representing the number of cells in the trophectoderm.

The tenth invention of the present application is directed to the embryo classification method of any of the first invention to the ninth invention, further including d) a learning step of creating the machine learning model by a machine learning algorithm using the parameter and a known classification result of the blastocyst embryo corresponding to the parameter, as training data.

The eleventh invention of the present application is directed to a computer program that causes a computer to perform the embryo classification method of any of the first invention to the tenth invention.

The twelfth invention of the present application is directed to an embryo classification apparatus for classifying a blastocyst embryo, including: an image acquisition unit configured to acquire a three-dimensional image of the blastocyst embryo by optical coherence tomography; a parameter calculation unit configured to calculate a parameter representing at least one of a volume of an inner cell mass, a thickness of the inner cell mass, a thickness distribution of the inner cell mass, a volume of a trophectoderm, a thickness of the trophectoderm, a thickness distribution of the trophectoderm, or the number of cells in the trophectoderm, on the basis of the three-dimensional image; and a classification unit configured to input the parameter of the blastocyst embryo to be classified, to a trained machine learning model that has the parameter as an input variable and has a classification result of the blastocyst embryo as an output variable, and classify the blastocyst embryo on the basis of the classification result output from the machine learning model.

### Advantageous Effects of Invention

According to the first invention to twelfth invention of the present application, classification of a blastocyst embryo that has conventionally been performed by an observer's subjective determination on the basis of a two-dimensional microscopic image can be performed quantitatively on the basis of a three-dimensional image.

### Brief Description of Drawings

Fig. 1 is a view showing a configuration of an embryo classification apparatus.
Fig. 2 is a control block diagram of the embryo classification apparatus.
Fig. 3 is a block diagram conceptually showing functions of a computer for classifying a blastocyst embryo.
Fig. 4 is a view showing a structure of the blastocyst embryo.
Fig. 5 is a flowchart showing a flow of a classification process of the blastocyst embryo.
Fig. 6 is a view showing multiple regions in a three-dimensional image.
Fig. 7 is a view showing an example of a histogram representing a thickness distribution.

### Description of Embodiments

Now, an embodiment of the present invention is described with reference to the drawings.

### <1. Configuration of Embryo Classification Apparatus>

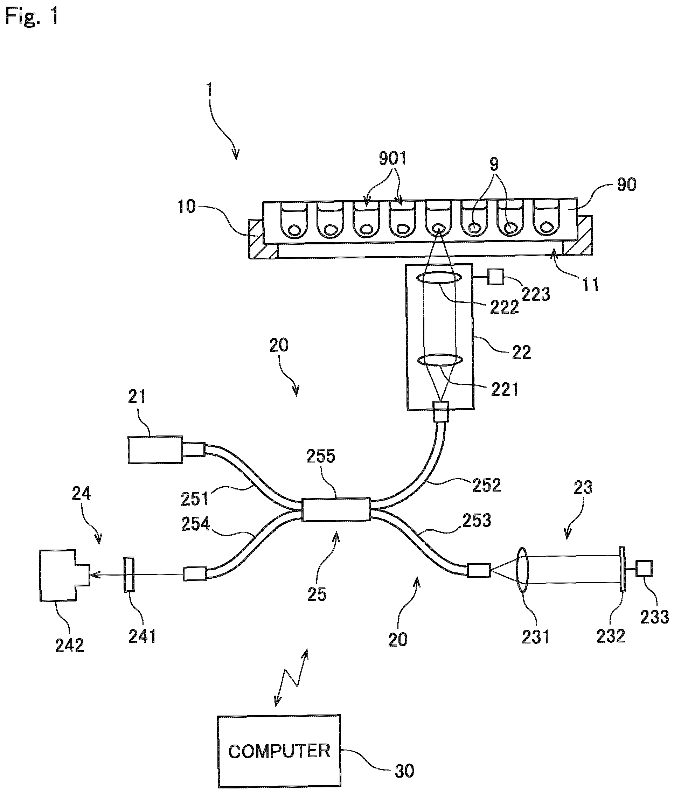

Fig. 1 is a view showing a configuration of an embryo classification apparatus 1 capable of performing a classification method according to one embodiment of the present invention. The embryo classification apparatus 1 is an apparatus that takes an image of a blastocyst embryo 9 held in a sample holder 90 by optical coherence tomography and classifies a state of the blastocyst embryo 9 on the basis of the thus acquired three-dimensional image. The blastocyst embryo 9 is a human embryo in one example, but may be a non-human animal's embryo.

As shown in Fig. 1, the embryo classification apparatus 1 includes a stage 10, an imaging unit 20, and a computer 30.

The stage 10 is a support frame that supports the sample holder 90. For the sample holder 90, for example, a well plate is used. The well plate includes multiple wells (recessed portions) 901. Each of the wells 901 has a U-shaped or V-shaped bottom. The blastocyst embryo 9, together with a culture medium, is held near the bottom of each well 901. For a material of the sample holder 90, transparent resin or glass that transmits light is used.

The stage 10 includes an opening 11 that vertically penetrates. The sample holder 90 is horizontally supported while being fit in the opening 11 of the stage 10. Thus, a lower surface of the sample holder 90 is exposed toward the imaging unit 20 without being covered by the stage 10.

The imaging unit 20 is a unit that takes an image of the blastocyst embryo 9 in the sample holder 90. The imaging unit 20 is placed below the sample holder 90 supported by the stage 10. The imaging unit 20 is an optical coherence tomography (OCT) device capable of acquiring a tomographic image and a three-dimensional image of the blastocyst embryo 9.

As shown in Fig. 1, the imaging unit 20 includes a light source 21, an object optical system 22, a reference optical system 23, a detection unit 24, and an optical fiber coupler 25. The optical fiber coupler 25 includes first to fourth optical fibers 251 to 254 connected at a connecting unit 255. The light source 21, the object optical system 22, the reference optical system 23, and the detection unit 24 are connected to each other via optical paths formed by the optical fiber coupler 25.

The light source 21 includes a light emitting element such as an LED. The light source 21 emits low-coherence light containing a wide range of wavelength components. In order to allow light to reach the inside of the blastocyst embryo 9 without invasively treating the blastocyst embryo 9, it is desirable that light emitted from the light source 21 is a near-infrared ray. The light source 21 is connected to the first optical fiber 251. Light emitted from the light source 21 is incident on the first optical fiber 251 and is separated into light incident on the second optical fiber 252 and light incident on the third optical fiber 253 at the connecting unit 255.

The second optical fiber 252 is connected to the object optical system 22. Light traveling from the connecting unit 255 to the second optical fiber 252 is incident on the object optical system 22. The object optical system 22 includes multiple optical components including a collimator lens 221 and an object lens 222. Light emitted from the second optical fiber 252 passes through the collimator lens 221 and the object lens 222, and is applied to the blastocyst embryo 9 in the sample holder 90. At that time, the object lens 222 causes the light to converge to the blastocyst embryo 9. Then, light (hereinafter referred to as "observation light") reflected from the blastocyst embryo 9 passes through the object lens 222 and the collimator lens 221 and is again incident on the second optical fiber 252.

As shown in Fig. 1, the object optical system 22 is connected to a scan mechanism 223. The scan mechanism 223 slightly moves the object optical system 22 vertically and horizontally in accordance with an instruction from the computer 30. Thus, an incidence position of light on the blastocyst embryo 9 can be slightly moved vertically and horizontally.

Further, the imaging unit 20 can be moved horizontally by a movement mechanism not shown. Thus, the field of view of the imaging unit 20 can be changed among the multiple wells 901.

The third optical fiber 253 is connected to the reference optical system 23. Light travelling from the connecting unit 255 to the third optical fiber 253 is incident on the reference optical system 23. The reference optical system 23 includes a collimator lens 231 and a mirror 232. Light emitted from the third optical fiber 253 passes through the collimator lens 231 and is incident on the mirror 232. Then, light (hereinafter referred to as "reference light") reflected from the mirror 232 passes through the collimator lens 231 and is again incident on the third optical fiber 253.

As shown in Fig. 1, the mirror 232 is connected to a retraction mechanism 233. The retraction mechanism 233 slightly moves the mirror 232 in an optical-axis direction in accordance with an instruction from the computer 30. Thus, an optical-path length of the reference light can be changed.

The fourth optical fiber 254 is connected to the detection unit 24. The observation light that is incident on the second optical fiber 252 from the object optical system 22 and the reference light that is incident on the third optical fiber 253 from the reference optical system 23 join together at the connecting unit 255, and are incident on the fourth optical fiber 254. Then, light emitted from the fourth optical fiber 254 is incident on the detection unit 24. At that time, interference is caused between the observation light and the reference light due to a phase difference therebetween. The optical spectrum of interference light at that time varies with a height of a position where the observation light is reflected.

The detection unit 24 includes a spectroscope 241 and a light detector 242. The interference light emitted from the fourth optical fiber 254 is dispersed into each wavelength component in the spectroscope 241, and is incident on the light detector 242. The light detector 242 detects each dispersed interference light, and outputs its corresponding detection signal to the computer 30.

An image acquisition unit 41 described later in the computer 30 performs Fourier transform on the detection signal provided from the light detector 242, to thereby calculate a vertical light-intensity distribution of the observation light. Further, while the object optical system 22 is horizontally moved by the scan mechanism 223, the image acquisition unit 41 repeats the above-described calculation of light-intensity distribution to thereby calculate a light-intensity distribution of the observation light at each coordinate position in a three-dimensional space. Consequently, the computer 30 can acquire a tomographic image and a three-dimensional image of the blastocyst embryo 9.

The tomographic image is formed of multiple pixels arranged on two-dimensional coordinates, and is data in which each pixel has a predetermined luminance value. The three-dimensional image is formed of multiple voxels arranged on three-dimensional coordinates and is data in which each voxel has a predetermined luminance value. That is, each of the tomographic image and the three-dimensional image is a taken image in which luminance values are distributed on predetermined coordinates.

The computer 30 functions as a control unit that controls an operation of the imaging unit 20. Further, the computer 30 functions as a data processing unit that produces a tomographic image and a three-dimensional image on the basis of a detection signal received from the imaging unit 20 and classifies the blastocyst embryo 9 on the basis of the acquired tomographic image and three-dimensional image.

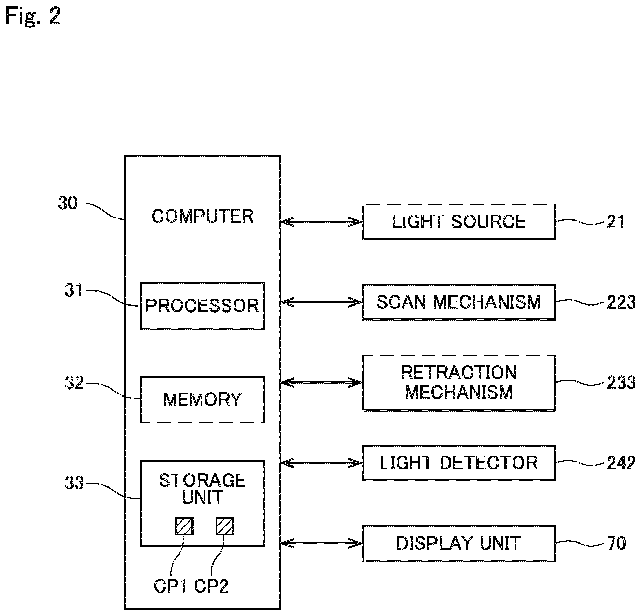

Fig. 2 is a control block diagram of the embryo classification apparatus 1. As conceptually shown in Fig. 2, the computer 30 includes a processor 31 such as a CPU, a memory 32 such as a RAM, and a storage unit 33 such as a hard disk drive. In the storage unit 33, a control program CP1 that is a computer program for controlling operations of respective components in the embryo classification apparatus 1 and a data processing program CP2 that is a computer program for producing a tomographic image D1 and a three-dimensional image D2 and classifying the blastocyst embryo 9, are stored.

The control program CP1 and the data processing program CP2 are read out from a storage medium such as a CD or DVD on which the computer 30 can perform a reading operation, and are stored in the storage unit 33. Alternatively, the control program CP1 and the data processing program CP2 may be downloaded to the computer 30 via a network.

Further, as shown in Fig. 2, the computer 30 is connected to the light source 21, the scan mechanism 223, the retraction mechanism 233, and the light detector 242 that have been described above such that the computer 30 can conduct communication with each of those components. Moreover, the computer 30 is connected also to a display unit 70 such as a liquid crystal display such that the computer 30 can conduct communication with the display unit 70. The computer 30 controls operations of the above-described respective components in accordance with the control program CP1. Thus, an image taking process of the blastocyst embryo 9 held in the sample holder 90 progresses.

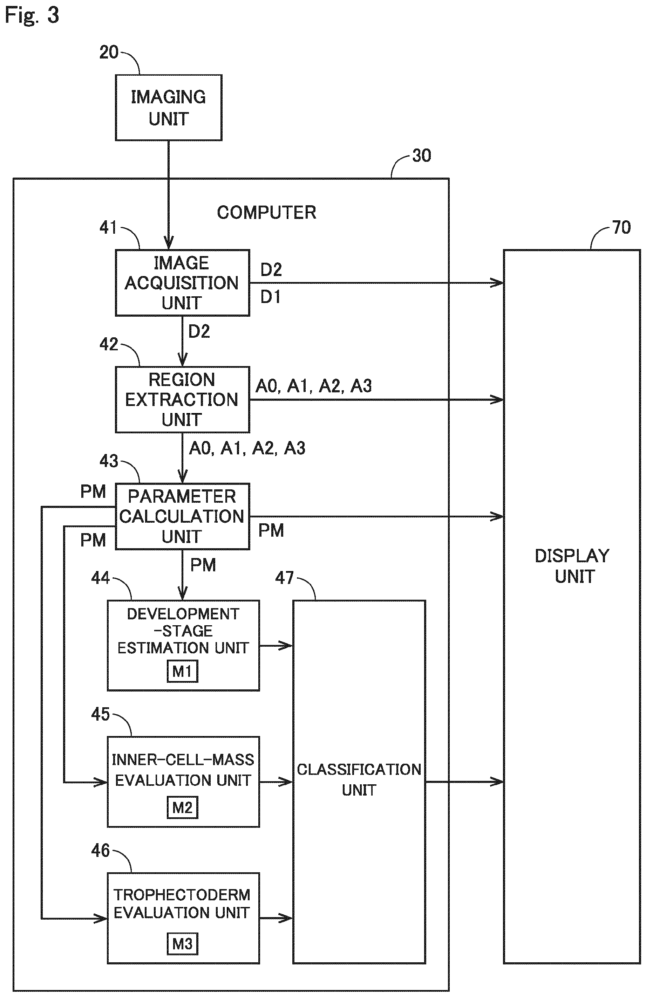

Fig. 3 is a block diagram conceptually showing functions of the computer 30 for classifying the blastocyst embryo 9. As shown in Fig. 3, the computer 30 includes the image acquisition unit 41, a region extraction unit 42, a parameter calculation unit 43, a development-stage estimation unit 44, an inner-cell-mass evaluation unit 45, a trophectoderm evaluation unit 46, and a classification unit 47. The respective functions of the image acquisition unit 41, the region extraction unit 42, the parameter calculation unit 43, the development-stage estimation unit 44, the inner-cell-mass evaluation unit 45, the trophectoderm evaluation unit 46, and the classification unit 47 are performed by an operation of the processor 31 of the computer 30 in accordance with the data processing program CP2 described above. Details of processes performed by the image acquisition unit 41, the region extraction unit 42, the parameter calculation unit 43, the development-stage estimation unit 44, the inner-cell-mass evaluation unit 45, the trophectoderm evaluation unit 46, and the classification unit 47 will be given later.

### <2. Classification Process of Blastocyst Embryo>

Next, a classification process of the blastocyst embryo 9 using the above-described embryo classification apparatus 1 is described.

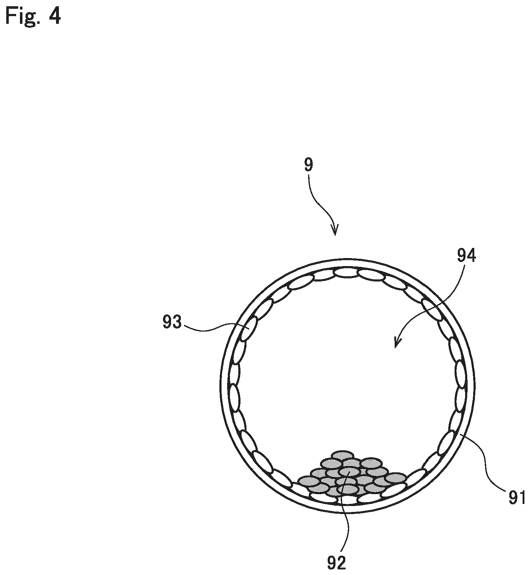

Fig. 4 is a view showing a structure of the blastocyst embryo 9. The blastocyst embryo 9 is an embryo at any stage from about five to seven days after fertilization until implantation. The embryo, after fertilization, goes through a cleavage stage, to become the blastocyst embryo 9. The blastocyst embryo 9 is transparent or semi-transparent. As shown in Fig. 4, the blastocyst embryo 9 includes a hollow spherical zona pellucida 91, and an inner cell mass 92 and a trophectoderm 93 that are enclosed in the zona pellucida 91. The inner cell mass (ICM) 92 is a part that develops to become a fetus. The trophectoderm (TE) 93 is a part that develops to become a placenta. The inner cell mass 92 and the trophectoderm 93 are each formed of multiple cells. Further, in the zona pellucida 91, a blastocyst cavity 94 surrounded by the inner cell mass 92 and the trophectoderm 93 is present.

The blastocyst embryo 9 goes through six development stages of a first early blastocyst, a second early blastocyst, a complete blastocyst, an expanded blastocyst, an escaped blastocyst, and a hatched blastocyst. In the first early blastocyst, a proportion of the blastocyst cavity 94 in the zona pellucida 91 is less than 50%. In the second early blastocyst, a proportion of the blastocyst cavity 94 in the zona pellucida 91 is 50% or more. In the complete blastocyst, a proportion of the blastocyst cavity 94 in the zona pellucida 91 is substantially maximized. In the expanded blastocyst, the blastocyst embryo 9 becomes larger and the zona pellucida 91 becomes thinner. In the escaped blastocyst, the inner cell mass 92 and the trophectoderm 93 are being hatched out of the zona pellucida 91. In the hatched blastocyst, hatching of the inner cell mass 92 and the trophectoderm 93 out of the zona pellucida 91 is completed.

The embryo classification apparatus 1 evaluates a development stage of the blastocyst embryo 9, a state of the inner cell mass 92, and a state of the trophectoderm 93, to thereby select the blastocyst embryo 9 that has been cultured successfully.

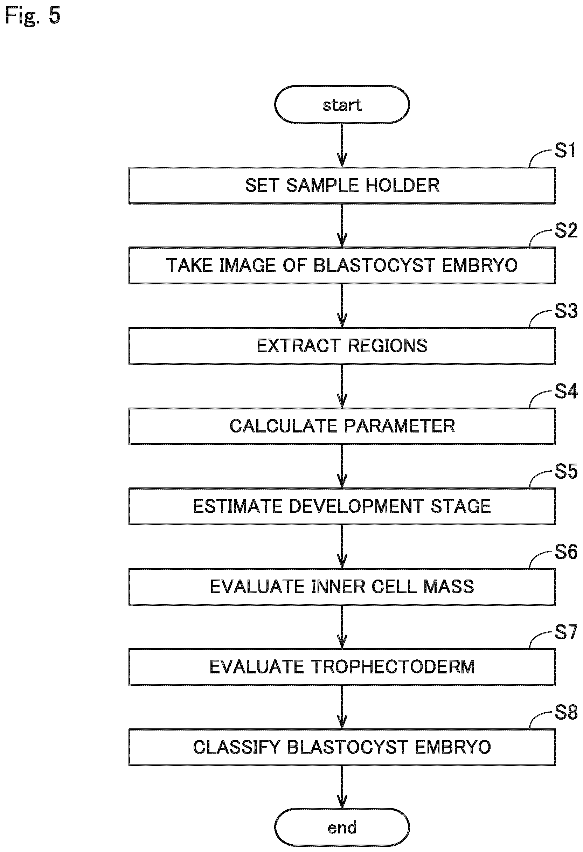

Fig. 5 is a flowchart showing a flow of a classification process of the blastocyst embryo 9. In classifying the blastocyst embryo 9 in the embryo classification apparatus 1, first, the sample holder 90 is set on the stage 10 (step S1, preparation step). In the sample holder 90, the blastocyst embryo 9 together with a culture medium is held.

Subsequently, in the embryo classification apparatus 1, an image of the blastocyst embryo 9 is taken by the imaging unit 20 (step S2, image acquisition step). The imaging unit 20 takes an image by optical coherence tomography. Specifically, the light source 21 is caused to emit light. Then, while the object optical system 22 is slightly moved by the scan mechanism 223, each wavelength component of interference light of observation light and reference light is detected by the light detector 242. The image acquisition unit 41 of the computer 30 calculates a light-intensity distribution at each coordinate position of the blastocyst embryo 9 on the basis of each detection signal output from the light detector 242. Consequently, the tomographic image D1 and the three-dimensional image D2 of the blastocyst embryo 9 are acquired.

The embryo classification apparatus 1 acquires multiple tomographic images D1 and one three-dimensional image D2 for one blastocyst embryo 9. Further, the embryo classification apparatus 1 repeats the process of the step S2 while changing the well 901 as an object of image taking, to thereby acquire the tomographic images D1 and the three-dimensional images D2 of the multiple blastocyst embryos 9. The acquired tomographic images D1 and three-dimensional images D2 are stored in the storage unit 33 of the computer 30. Moreover, the computer 30 displays the acquired tomographic images D1 and three-dimensional images D2 on the display unit 70.

Then, the region extraction unit 42 of the computer 30 extracts multiple regions in the three-dimensional image D2 of the blastocyst embryo 9 (step S3, region extraction step). Specifically, the region extraction unit 42 extracts each of an entire embryo region A0 corresponding to the entire blastocyst embryo 9, a zona-pellucida region A1 corresponding to the zona pellucida 91, an ICM region A2 corresponding to the inner cell mass 92, and a TE region A3 corresponding to the trophectoderm 93 in the three-dimensional image D2 of the blastocyst embryo 9.

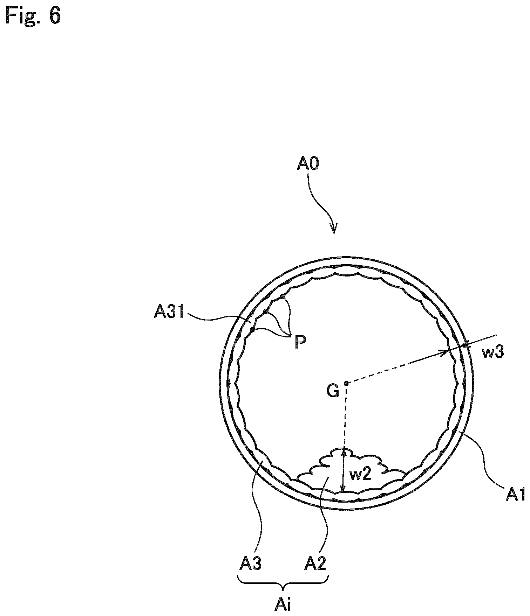

Fig. 6 is a view showing the multiple regions extracted in the step S3. As shown in Fig. 6, the region extraction unit 42 extracts the entire embryo region A0 from the three-dimensional image D2, first. Secondly, the region extraction unit 42 extracts a substantially hollow spherical region having a predetermined thickness measured from an outer surface of the entire embryo region A0, as the zona-pellucida region A1. Subsequently, the region extraction unit 42 specifies a remaining region (hereinafter referred to as an "inner region Ai") resulting from excluding the zona-pellucida region A1 from the entire embryo region A0. The inner region Ai includes the ICM region A2 and the TE region A3.

Subsequently, the region extraction unit 42 calculates a thickness of a part having a relatively small thickness along a radial direction centered at a centroid G in the inner region Ai, as a thickness of the TE region A3. Then, the region extraction unit 42 extracts a remaining region resulting from excluding a substantially hollow spherical region having a thickness corresponding to the thickness of the TE region A3 from an outer surface of the inner region Ai, as the ICM region A2. After that, the region extraction unit 42 extracts a remaining region resulting from excluding the ICM region A2 from the inner region Ai, as the TE region A3.

The computer 30 stores the entire embryo region A0, the zona-pellucida region A1, the ICM region A2, and the TE region A3 that are extracted by the region extraction unit 42, into the storage unit 33. Further, the computer 30 may display the zona-pellucida region A1, the ICM region A2, and the TE region A3 while performing color coding or the like on each region in the three-dimensional image D2 displayed on the display unit 70.

Subsequently, the parameter calculation unit 43 of the computer 30 calculates multiple parameters PM on the basis of each of the above-described regions A0, A1, A2, and A3 (step S4, parameter calculation step). The multiple parameters PM include a parameter PM representing at least one of the following (1) to (7).
(1) Volume of the inner cell mass 92
(2) Thickness of the inner cell mass 92
(3) Thickness distribution of the inner cell mass 92
(4) Volume of the trophectoderm 93
(5) Thickness of the trophectoderm 93
(6) Thickness distribution of the trophectoderm 93
(7) Number of cells in the trophectoderm 93

### (1) Volume of the inner cell mass 92

The parameter calculation unit 43 calculates the parameter PM representing a volume of the inner cell mass 92 on the basis of the above-described ICM region A2. For example, the parameter calculation unit 43 calculates the number of voxels forming the ICM regions A2 as the parameter PM representing the volume of the inner cell mass 92. Note that the parameter calculation unit 43 may calculate the volume of the inner cell mass 92 in the real space by multiplying a volume per voxel in the real space by the number of voxels forming the ICM region A2.

### (2) Thickness of the inner cell mass 92

The parameter calculation unit 43 calculates the parameter PM representing a thickness of the inner cell mass 92 on the basis of the above-described ICM region A2. For example, as shown in Fig. 6, the parameter calculation unit 43 calculates a thickness w2 of the ICM region A2 along a radial direction centered at a centroid G of the entire embryo region A0, as the parameter PM representing the thickness of the inner cell mass 92. Further, the parameter calculation unit 43 may calculate thicknesses w2 of the ICM region A2 at multiple points, and calculate at least one of an average value, a mean value, a standard deviation, a minimum value, a maximum value, or a range, of the thicknesses, as the parameter PM representing the thickness of the inner cell mass 92.

### (3) Thickness distribution of the inner cell mass 92

The parameter calculation unit 43 calculates the parameter PM representing a thickness distribution of the inner cell mass 92 on the basis of the above-described ICM region A2. For example, the parameter calculation unit 43 collects the above-described thicknesses w2 of the ICM region A2 at multiple points, and calculates at least one of kurtosis, skewness, or a mode value, of the thicknesses, as the parameter PM representing the thickness distribution of the inner cell mass 92.

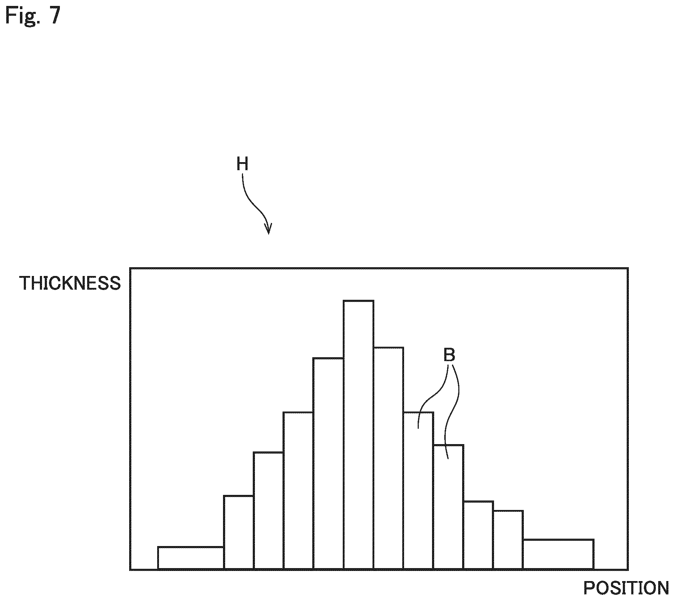

Alternatively, the parameter calculation unit 43 may create a histogram H on the basis of the thicknesses w2 of the ICM region A2 at multiple points, as shown in Fig. 7. Then, the parameter calculation unit 43 may calculate a density of each bin B of the histogram H in the entirety, as the parameter PM representing the thickness distribution of the inner cell mass 92.

### (4) Volume of the trophectoderm 93

The parameter calculation unit 43 calculates the parameter PM representing a volume of the trophectoderm 93 on the basis of the above-described TE region A3. For example, the parameter calculation unit 43 calculates the number of voxels forming the TE region A3, as the parameter PM representing the volume of the trophectoderm 93. Note that the parameter calculation unit 43 may calculate the volume of the trophectoderm 93 in the real space by multiplying a volume per voxel in the real space by the number of voxels forming the TE region A3.

### (5) Thickness of the trophectoderm 93

The parameter calculation unit 43 calculates the parameter PM representing a thickness of the trophectoderm 93 on the basis of the above-described TE region A3. For example, as shown in Fig. 6, the parameter calculation unit 43 calculates a thickness w3 of the TE region A3 along a radial direction centered at the centroid G of the entire embryo region A0, as the parameter PM representing the thickness of the trophectoderm 93. Further, the parameter calculation unit 43 may calculate thicknesses w3 of the TE region A3 at multiple points, and calculate at least one of an average value, a mean value, a standard deviation, a minimum value, a maximum value, or a range, of the thicknesses, as the parameter PM representing the thickness of the trophectoderm 93.

### (6) Thickness distribution of the trophectoderm 93

The parameter calculation unit 43 calculates the parameter PM representing a thickness distribution of the trophectoderm 93 on the basis of the above-described TE region A3. For example, the parameter calculation unit 43 collects the above-described thicknesses w3 of the TE region A3 at multiple points, and calculates at least one of kurtosis, skewness, or a mode value, of the thicknesses, as the parameter PM representing the thickness distribution of the trophectoderm 93.

Alternatively, the parameter calculation unit 43 may create the histogram H like that in Fig. 7, on the basis of the thicknesses w3 of the TE region A3 at multiple points. Then, the parameter calculation unit 43 may calculate a density of each bin B of the histogram H in the entirety, as the parameter PM representing the thickness distribution of the trophectoderm 93.

### (7) The number of cells in the trophectoderm 93

The parameter calculation unit 43 calculates the parameter PM representing the number of cells in the trophectoderm 93 on the basis of the above-described TE region A3. For example, the parameter calculation unit 43 calculates the number of peak points P of the thickness w3 of a part (hereinafter referred to as a "non-overlapping TE region A31") of the TE region A3 not radially overlapping the ICM region A2. Then, the parameter calculation unit 43 calculates the parameter PM representing the number of cells in the trophectoderm 93 on the basis of the number of the peak points P.

However, the number of the peak points P in the non-overlapping TE region A31 does not reflect the number of cells in a part of the trophectoderm 93 overlapping the inner cell mass 92. For this reason, the parameter calculation unit 43 may calculate a cell density of the trophectoderm 93 by dividing the number of cells calculated from the number of the above-described peak points P by a volume of the non-overlapping TE region A31. Then, the calculated cell density may be used as the parameter PM representing the number of cells in the trophectoderm 93.

Alternatively, the parameter calculation unit 43 may calculate the parameter PM representing the number of cells in the entire trophectoderm 93 by multiplying the above-described cell density by the volume of the TE region A3.

The computer 30 stores the multiple parameters PM calculated by the parameter calculation unit 43 into the storage unit 33. Further, the computer 30 may display the multiple parameters PM calculated by the parameter calculation unit 43 on the display unit 70.

Subsequently, the development-stage estimation unit 44 of the computer 30 evaluates a development stage of the blastocyst embryo 9 (step S5). In the storage unit 33 of the computer 30, a development-stage estimation model M1 for estimating a development stage of the blastocyst embryo 9 is stored. The development-stage estimation model M1 is a trained machine learning model that has at least one of the above-described multiple parameters PM as an input variable and has an estimation result of a development stage as an output variable.

The development-stage estimation model M1 is created in advance by machine learning using a supervised machine learning algorithm. For the machine learning, many combinations each including the above-described parameter PM and a known development stage of the blastocyst embryo 9 corresponding to the parameter PM are prepared. Then, a learning model is updated by the supervised machine learning algorithm using the combinations as training data. This results in creation of the development-stage estimation model M1 that can accurately output a development stage of the blastocyst embryo 9 on the basis of the input parameter PM.

In the step S5, the development-stage estimation unit 44 inputs the parameter PM calculated in the step S4 to the development-stage estimation model M1. As a result, an estimation result of a development stage of the blastocyst embryo 9 is output from the development-stage estimation model M1.

The development-stage estimation unit 44 estimates a development stage of the blastocyst embryo 9 to be any of the six stages of the first early blastocyst, the second early blastocyst, the complete blastocyst, the expanded blastocyst, the escaped blastocyst, and the hatched blastocyst according to Gardner's classification. Note that the classification of a development stage is not necessarily required to agree with Gardner's classification.

Subsequently, the inner-cell-mass evaluation unit 45 of the computer 30 evaluates a state of the inner cell mass 92 (step S6). In the storage unit 33 of the computer 30, an inner-cell-mass evaluation model M2 for evaluating a state of the inner cell mass 92 is stored. The inner-cell-mass evaluation model M2 is a trained machine learning model that has at least one of the above-described multiple parameters PM as an input variable and has an evaluation result of the inner cell mass 92 as an output variable.

The inner-cell-mass evaluation model M2 is created in advance by machine learning using a supervised machine learning algorithm. For the machine learning, many combinations each including the above-described parameter PM and a known evaluation result of the inner cell mass 92 corresponding to the parameter PM are prepared. Then, a learning model is updated by the supervised machine learning algorithm using the combinations as training data. This results in creation of the inner-cell-mass evaluation model M2 that can accurately output an evaluation result of the inner cell mass 92 on the basis of the input parameter PM.

In the step S6, the inner-cell-mass evaluation unit 45 inputs the parameter PM calculated in the step S4 to the inner-cell-mass evaluation model M2. As a result, an evaluation result of the inner cell mass 92 of the blastocyst embryo 9 is output from the inner-cell-mass evaluation model M2.

The inner-cell-mass evaluation unit 45 evaluates a state of the inner cell mass 92 to be at any of three stages A to C according to Gardner's classification. A represents that the number of cells in the inner cell mass 92 is large and the cells are dense. B represents that the number of cells in the inner cell mass 92 is smaller than A and the cells are sparse. C represents that few cells are found in the inner cell mass 92. Note that the evaluation criteria for the inner cell mass 92 is not necessarily required to agree with Gardner's classification.

Subsequently, the trophectoderm evaluation unit 46 of the computer 30 evaluates a state of the trophectoderm 93 (step S7). In the storage unit 33 of the computer 30, a trophectoderm evaluation model M3 for evaluating a state of the trophectoderm 93 is stored. The trophectoderm evaluation model M3 is a trained machine learning model that has at least one of the above-described multiple parameters PM as an input variable and has an evaluation result of the trophectoderm 93 as an output variable.

The trophectoderm evaluation model M3 is created in advance by machine learning using a supervised machine learning algorithm. For the machine learning, many combinations each including the above-described parameter PM and a known evaluation result of the trophectoderm 93 corresponding to the parameter PM are prepared. Then, a learning model is updated by the supervised machine learning algorithm using the combinations as training data. This results in creation of the trophectoderm evaluation model M3 that can accurately output an evaluation result of the trophectoderm 93 on the basis of the input parameter PM.

In the step S7, the trophectoderm evaluation unit 46 inputs the parameter PM calculated in the step S4 to the trophectoderm evaluation model M3. As a result, an evaluation result of the trophectoderm 93 of the blastocyst embryo 9 is output from the trophectoderm evaluation model M3.

The trophectoderm evaluation unit 46 evaluates a state of the trophectoderm 93 to be at any of three stages A to C according to Gardner's classification. A represents that the number of cells in the trophectoderm 93 is large and the cells are dense. B represents that the number of cells in the trophectoderm 93 is smaller than A and the cells are sparse. C represents that the number of cells in the trophectoderm 93 is smaller than B and the cells are large in size. Note that the evaluation criteria for the trophectoderm 93 is not necessarily required to agree with Gardner's classification.

Note that the computer 30 may perform the steps S5 to S7 in an order different from the above-described order. Alternatively, the computer 30 may perform the steps S5 to S7 simultaneously.

After that, the classification unit 47 of the computer 30 classifies the blastocyst embryo 9 on the basis of the results provided by the above-described steps S5 to S7 (step S8, classification step). For example, in a case in which a development stage of the blastocyst embryo 9 is estimated to be any of the six stages in the step S5, a state of the inner cell mass 92 is evaluated to be at any of the three stages in the step S6, and a state of the trophectoderm 93 is evaluated to be at any of the three stages in the step S7, the classification unit 47 can classify the blastocyst embryo 9 as any of 6 × 3 × 3 = 54 groups, using the results. That is, in the present embodiment, the estimation result of a development stage, the evaluation result of the inner cell mass 92, and the evaluation result of the trophectoderm 93 that have been described above are each used as a classification result.

The computer 30 stores the classification result of the blastocyst embryo 9 into the storage unit 33. Further, the computer 30 displays the classification result of the blastocyst embryo 9 on the display unit 70.

As described above, in the present embodiment, the computer 30 calculates the multiple parameters PM regarding the blastocyst embryo 9 on the basis of the three-dimensional image D2. Then, the computer 30 inputs the parameters PM to a machine learning model, and classifies the blastocyst embryo 9 on the basis of a classification result output from the machine learning model. Hence, classification of the blastocyst embryo 9 that has conventionally been performed by an observer's subjective determination on the basis of a two-dimensional microscopic image can be performed quantitatively on the basis of the three-dimensional image D2. This makes it possible to obtain an accurate classification result based on the three-dimensional image D2. Therefore, use of the classification method of the present embodiment can improve a performance rate such as an implantation rate and a pregnancy rate in an assisted reproductive technology procedure. Further, it is possible to reduce burdens on an embryologist and obtain an objective classification result of the blastocyst embryo 9 not depending on skills of an embryologist.

### <3. Modifications>

Hereinabove, one embodiment of the present invention has been described, but the present invention is not limited to the above-described embodiment.

In the above-described embodiment, the classification unit 47 classifies a development stage of the blastocyst embryo 9 into six stages, classifies a state of the inner cell mass 92 into three stages, and classifies a state of the trophectoderm 93 into three stages. Alternatively, the classification unit 47 may classify the blastocyst embryo 9 in another way. For example, the classification unit 47 may classify only one or two among a development stage of the blastocyst embryo 9, a state of the inner cell mass 92, and a state of the trophectoderm 93. Further alternatively, the classification unit 47 may classify the blastocyst embryos 9 into only two types of a "good embryo" and a "not-good embryo" on the basis of the parameter PM calculated from the three-dimensional image D2.

Further, in the above-described embodiment, the parameter PM regarding the blastocyst embryo 9 is calculated on the basis of only the three-dimensional image D2 acquired by optical coherence tomography. Alternatively, the parameter calculation unit 43 may calculate the parameter PM regarding the blastocyst embryo 9 on the basis of the tomographic image D1 and the three-dimensional image D2 that are acquired by optical coherence tomography. Further alternatively, the parameter calculation unit 43 may calculate the parameter PM regarding the blastocyst embryo 9 on the basis of the three-dimensional image D2 acquired by optical coherence tomography and a microscopic image additionally acquired.

Further, the parameter PM input to the machine learning model is not limited to the above-described (1) to (7). For example, a volume of the entire blastocyst embryo 9, a thickness of the zona pellucida 91, or the like may be added as the parameter PM.

In the above-described embodiment, the sample holder 90 is a well plate including the multiple wells (recessed portions) 901, and each of the wells 901 holds one blastocyst embryo 9. However, the sample holder 90 that holds the blastocyst embryo 9 is not limited to a well plate. For example, the sample holder 90 may be a dish having only one recessed portion.

Moreover, the respective elements described in the above-described embodiment and modifications may be appropriately combined unless contradiction occurs.

### Reference Signs List

- 1: Embryo classification apparatus
- 9: Blastocyst embryo
- 10: Stage
- 20: Imaging unit
- 30: Computer
- 41: Image acquisition unit
- 42: Region extraction unit
- 43: Parameter calculation unit
- 44: Development-stage estimation unit
- 45: Inner-cell-mass evaluation unit
- 46: Trophectoderm evaluation unit
- 47: Classification unit
- 70: Display unit
- 90: Sample holder
- 91: Zona pellucida
- 92: Inner cell mass
- 93: Trophectoderm
- A0: Entire embryo region
- A1: Zona-pellucida region
- A2: ICM region
- A3: TE region
- CP 1: Control program
- CP2: Data processing program
- D1: Tomographic image
- D2: Three-dimensional image
- G: Central point
- H: Histogram
- M1: Development-stage estimation model
- M2: Inner-cell-mass evaluation model
- M3: Trophectoderm evaluation model
- PM: Parameter

## Claims

1. An embryo classification method for classifying a blastocyst embryo, comprising:
a) an image acquisition step of acquiring a three-dimensional image of the blastocyst embryo by optical coherence tomography;
b) a parameter calculation step of calculating a parameter representing at least one of a volume of an inner cell mass, a thickness of the inner cell mass, a thickness distribution of the inner cell mass, a volume of a trophectoderm, a thickness of the trophectoderm, a thickness distribution of the trophectoderm, or the number of cells in the trophectoderm, on the basis of the three-dimensional image; and
c) a classification step of inputting the parameter of the blastocyst embryo to be classified, to a trained machine learning model that has the parameter as an input variable and has a classification result of the blastocyst embryo as an output variable, and classifying the blastocyst embryo on the basis of the classification result output from the machine learning model.

2. The embryo classification method according to claim 1, wherein the classification result includes an evaluation result of the inner cell mass.

3. The embryo classification method according to claim 1, wherein the classification result includes an evaluation result of the trophectoderm.

4. The embryo classification method according to claim 1, wherein the classification result includes an estimation result of a development stage of the blastocyst embryo.

5. The embryo classification method according to any of claims 1 to 4, wherein the input variable includes at least one of an average value, a mean value, a standard deviation, a minimum value, a maximum value, or a range, of the thickness of the inner cell mass, as the parameter representing the thickness of the inner cell mass.

6. The embryo classification method according to any of claims 1 to 5, wherein the input variable includes at least one of kurtosis, skewness, a mode value, or a density of each bin of a histogram, of the thickness distribution of the inner cell mass, as the parameter representing the thickness distribution of the inner cell mass.

7. The embryo classification method according to any of claims 1 to 6, wherein the input variable includes at least one of an average value, a mean value, a standard deviation, a minimum value, a maximum value, or a range, of the thickness of the trophectoderm, as the parameter representing the thickness of the trophectoderm.

8. The embryo classification method according to any of claims 1 to 7, wherein the input variable includes at least one of kurtosis, skewness, a mode value, or a density of each bin of a histogram, of the thickness distribution of the trophectoderm, as the parameter representing the thickness distribution of the trophectoderm.

9. The embryo classification method according to any of claims 1 to 8, wherein the input variable includes the number of cells, or a cell density, of a part of the trophectoderm not overlapping the inner cell mass, as the parameter representing the number of cells in the trophectoderm.

10. The embryo classification method according to any of claims 1 to 9, further comprising d) a learning step of creating the machine learning model by a machine learning algorithm using the parameter and a known classification result of the blastocyst embryo corresponding to the parameter, as training data.

11. A computer program that causes a computer to perform the embryo classification method according to any of claims 1 to 10.

12. An embryo classification apparatus for classifying a blastocyst embryo, comprising:
an image acquisition unit configured to acquire a three-dimensional image of the blastocyst embryo by optical coherence tomography;
a parameter calculation unit configured to calculate a parameter representing at least one of a volume of an inner cell mass, a thickness of the inner cell mass, a thickness distribution of the inner cell mass, a volume of a trophectoderm, a thickness of the trophectoderm, a thickness distribution of the trophectoderm, or the number of cells in the trophectoderm, on the basis of the three-dimensional image; and
a classification unit configured to input the parameter of the blastocyst embryo to be classified, to a trained machine learning model that has the parameter as an input variable and has a classification result of the blastocyst embryo as an output variable, and classify the blastocyst embryo on the basis of the classification result output from the machine learning model.
